Europäisches Patentamt

European Patent Office

Office européen des brevets

(19)

(11) Numéro de publication: **0 468 872 B1**

# (12) FASCICULE DE BREVET EUROPEEN

(45) Date de publication du fascicule du brevet: **13.09.95**    (51) Int. Cl.⁶: **G01F 15/04**, G01N 33/22, G01N 1/22

(21) Numéro de dépôt: **91402032.6**

(22) Date de dépôt: **22.07.91**

---

(54) **Dispositif de comptage de gaz combustible.**

---

(30) Priorité: **23.07.90 FR 9009365**

(43) Date de publication de la demande:
**29.01.92 Bulletin 92/05**

(45) Mention de la délivrance du brevet:
**13.09.95 Bulletin 95/37**

(84) Etats contractants désignés:
**BE DE GB NL**

(56) Documents cités:
EP-A- 0 326 494     EP-A- 0 373 965
FR-A- 704 708       FR-A- 1 506 312
FR-A- 1 549 104     US-A- 4 396 299

(73) Titulaire: **COMPAGNIE FRANCAISE DU ME-
THANE
15 rue de Lubeck
F-75116 Paris (FR)**

(72) Inventeur: **Tachoires, Patrick
15, rue du Montier
F-95300 Ennery (FR)**

(74) Mandataire: **Boillot, Marc
ELF AOUITAINE
Division Propriété Industrielle
Tour Elf
F-92078 Paris la Défense Cédex 45 (FR)**

---

## Description

La présente invention concerne un dispositif de comptage, en terme de quantité d'énergie, de gaz combustible.

Les gaz, tel que le gaz naturel, les gaz de pétrole ou les gaz de cokerie, sont utilisés essentiellement comme combustibles. Ils peuvent être livrés en vrac sur le site de consommation. Plus souvent, les sociétés distributrices ont installé entre points de production et points de consommation sur tous leurs territoires des réseaux de distribution de gaz sous pression sur lesquels les consommateurs viennent prélever au fur et à mesure de leurs besoins les quantités qui leur sont nécessaires. Se pose alors le problème du comptage de ces quantités de gaz.

Pour les gaz dont on utilise les propriétés combustibles, ce qui est en fait utile et doit être facturé n'est pas le volume mais la quantité effective d'énergie qui aura été fournie par le distributeur. Il faut donc connaître les consommations en termes d'énergie.

La solution adoptée à l'heure actuelle est de placer au point de livraison un compteur qui totalise les volumes de gaz prélevés. S'il s'agit d'un petit consommateur, on se contente de multiplier le volume ainsi obtenu par un coefficient tenant compte de la température, de la pression et du pouvoir calorifique moyens du gaz livré.

S'il s'agit d'un consommateur moyen, afin d'améliorer la précision, le comptage sera constitué, en plus du compteur volumétrique, d'un transducteur de pression et d'un transducteur de température afin de déterminer les volumes corrigés livrés sur le site, en fait les masses de gaz effectivement consommées. Bien entendu cette correction augmente le prix de revient de l'installation. Il est toujours nécessaire, pour passer des masses aux quantités d'énergie de multiplier lesdites masses par un coefficient tenant compte du pouvoir calorifique du gaz. Ce genre de comptage serait précis si le pouvoir calorifique en question était parfaitement connu et constant. Ce n'est pas le cas d'où l'apparition d'une erreur plus ou moins acceptable due au fait que l'on utilise un pouvoir calorifique moyen, mesuré de façon discontinue, sur un site qui n'est pas généralement le site de livraison.

S'il s'agit de très gros échanges et que l'on désire une grande précision de mesure des quantités d'énergie fournies, on installe sur le site, en plus du comptage volumétrique corrigé en température et pression, un laboratoire qui mesure en continu les caractéristiques du gaz transité, c'est à dire au minimum le pouvoir calorifique supérieur et la densité. L'ensemble est alors piloté par un ou plusieurs organes de calcul. Une telle solution donne bien sûr des mesures de grande précision mais nécessite la mise en oeuvre de moyens matériels, humains et financiers importants.

La demande de brevet européen 0 326 494 décrit un procédé et un dispositif de mesurage de la puissance calorifique véhiculées par un courant de matière combustible. Le procédé consiste à prélever en continu une faible fraction connue du débit massique et à faire brûler le gaz ainsi dérivé pour mesurer ses caractéristiques. Le dispositif qui met en oeuvre ce procédé, comporte un échantillonneur pour prélever la fraction du gaz à faire brûler, un analyseur pour mesurer les éléments nécessaires à la détermination de la quantité d'énergie correspondant à cette fraction de gaz et un dispositif de calcul qui détermine, enregistre et affiche la puissance calorifique véhiculée par le courant de matière combustible. C'est une amélioration des méthodes précédentes, malheureusement, les compteurs utilisés industriellement mesurent le débit volumétrique des gaz livrés et non pas le débit massique.

Le problème à résoudre est donc de trouver un moyen plus simple permettant d'assurer le comptage de l'énergie fournie par un gaz combustible dans de bonnes conditions de précision et qui soit indépendant des conditions de température et de pression sans être obligé d'installer sur place un laboratoire de mesure complexe et onéreux.

La présente invention a précisément pour objet de résoudre ce problème. A cet effet, elle propose un dispositif de comptage des quantités d'énergie fournies par un réseau de distribution de gaz combustible du type comportant, sur la conduite, au point de livraison, un organe de comptage du volume brut de gaz livré et un échantillonneur qui prélève en permanence, aux conditions de température et de pression de la conduite, des volumes de gaz déterminés et un analyseur qui mesure les éléments nécessaires à la détermination des quantités d'énergie correspondant auxdits volumes. Un calculateur traite l'ensemble des informations et ramène en permanence lesdites quantités aux volumes bruts livrés pendant le même temps. Le calculateur intègre alors les résultats et donne les quantités totales d'énergie fournies par le réseau au point de distribution. Le dispositif se caractérisé en ce que l'échantillonneur se compose d'un cylindre pourvu d'un piston séparant ledit cylindre en deux zones, une zone étant alimentée par le réseau tandis que l'autre alimente l'analyseur, et de deux électrovannes trois voies commandées par le déplacement du piston et qui assurent les mises en communication des zones alternativement avec le réseau et avec l'analyseur

2

L'échantillonneur, placé sur la conduite de livraison à proximité du compteur volumétrique, est enrobé d'un circuit thermique de façon que le gaz soit prélevé non seulement à une pression identique à celle qui servira au comptage volumétrique principal mais aussi aux mêmes conditions de température. Pour améliorer encore l'identité des températures entre le gaz du réseau principal et le gaz prélevé, l'échantillonneur est placé directement dans la conduite principale de livraison. L'échantillonneur est conçu de telle façon qu'il assure en permanence le prélèvement de volumes fixes de gaz sur la conduite de livraison et l'envoi de ces volumes de gaz vers l'analyseur.

A la sortie de l'échantillonneur, le déversement vers l'analyseur est régulé en pression de façon que ce dernier ne soit pas affecté par les variations de pression qui peuvent intervenir sur le réseau de distribution.

L'analyseur permet de calculer la quantité de chaleur dégagée par la combustion de l'échantillon aux conditions du moment, c'est à dire l'énergie que dégage l'unité de volume de gaz lorsqu'on le brûle. Puisque l'on connait pendant le même temps le volume de gaz mesuré au compteur principal il est facile de calculer la quantité d'énergie correspondante et, en intégrant, les quantités d'énergie livrées en permanence.

Un avantage du dispositif objet de l'invention est de mesurer en permanence, dans les conditions même de la livraison, les quantités d'énergie fournie si bien que celles-ci ne dépendent ni de la nature chimique du gaz ni des conditions de température et de pression.

Un calculateur prend en compte en permanence les valeurs du débit d'air à l'analyseur, du débit volumétrique de gaz au point de livraison et délivre ainsi les quantités d'énergie fournies au consommateur.

D'autres buts, caractéristiques et avantages apparaîtront à la lecture de la description de divers modes de réalisation de l'invention, faite à titre non limitatif et en regard des dessins annexés, où
- la figure 1 représente une vue d'ensemble du dispositif,
- la figure 2 représente un schéma de principe d'un échantillonneur,

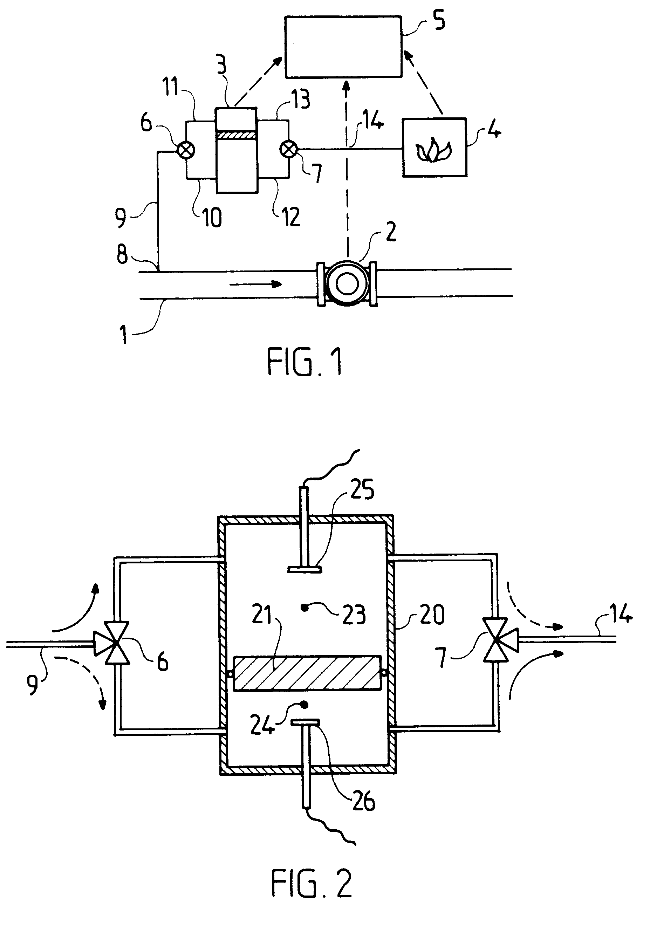

La figure 1 montre schématiquement l'ensemble du dispositif objet de l'invention. Sur la conduite réseau (1) est placé un système classique de comptage volumétrique de gaz (2). Il n'est demandé à ce comptage aucune caractéristique particulière du fait de l'invention si ce n'est qu'il doit être équipé d'une sortie, accessible par un calculateur, donnant en particulier le débit instantané de gaz. Les autres caractéristiques du compteur (2) seront déterminées par l'homme du métier en fonction des conditions de livraison telles que débit maximum, pression, température.

A proximité du compteur (2) un piquage (8) pour un capillaire (9) alimente une voie d'une vanne trois voies (6). La vanne (6) peut occuper deux positions, l'une mettant le capillaire (9) en communication avec le capillaire (10) et fermant le capillaire (11), l'autre faisant communiquer (9) avec (11) et fermant (10). Les capillaires (10) et (11) sont reliés à un échantillonneur (3). A la sortie de cet échantillonneur deux capillaires (12) et (13) sont reliés à une vanne trois voies (7). Elle peut occuper deux positions, l'une mettant un capillaire (14) en communication avec (12), l'autre mettant (14) en communication avec (13). Le capillaire (14) alimente l'analyseur (4). Le gaz prélevé sur la conduite (1) est donc envoyé, via l'échantillonneur (3), à l'analyseur (4).

La figure (2) montre le fonctionnement de l'échantillonneur (3). Il est composé d'un cylindre (20) à l'intérieur duquel se déplace un piston (21). Le gaz en provenance du réseau est conduit par le capillaire (9) alternativement vers la partie (23) et la partie (24) du cylindre en fonction de la position de la vanne (6). A la sortie de l'échantillonneur, le gaz est dirigé vers l'analyseur via la vanne trois voies (7) depuis les parties (23) ou (24) du cylindre (20). Les vannes (6) et (7) sont disposées de façon telle que si (9) et (23) sont mis en communication via la vanne (6), (24) et (14) sont mis en communication via la vanne (7) et vice versa.

A un moment donné, correspondant aux flèches en trait plein sur la figure (2), le gaz en provenance du réseau pousse le piston (21) et refoule le gaz contenu en (24) vers l'analyseur. Après un certain temps, le piston (21) arrive au contact d'un fin de course (26) qui inverse la position des deux électrovannes (6) et (7). Le mouvement du gaz s'inverse alors comme l'indiquent les flèches en pointillé sur la figure (2). Le mouvement du piston s'inverse également jusqu'à atteindre le fin de course (25). A ce moment, les vannes (6) et (7) sont à nouveau inversées et le cycle recommence. Le mouvement entre les deux fins de course (25) et (26) représente donc l'envoi de volumes fixes de gaz vers l'analyseur.

Le fonctionnement est donc tel que pendant un cycle N où l'on mesure un volume $V_N$ au compteur (2), l'échantillon analysé est celui prélevé au cycle précédent. La valeur $e_N$ de l'énergie de l'échantillon correspondant au prélèvement $V_N$ sera donné au cycle suivant pendant que l'on mesure $V_{N+1}$.

L'échantillonneur (3) est placé sur la conduite principale (1) et enrobé d'un ciment thermique maintenu par une coquille afin que le gaz soit prélevé aux conditions de températures de la conduite. Avantageusement, un calorifuge classique complète ce dispositif.

L'analyseur utilisé peut être, avantageusement, un appareil fabriqué par la société FLUID-DATA, type CAL 2000, société représentée en France par la société FLUIDYSTEME. Le CAL 2000 est un appareil qui peut mesurer soit le PCI soit l'indice de Wobbe. Le principe de base est le suivant : un débit de gaz régulier, en provenance de l'échantillonneur, arrive à un brûleur. Parallèlement, on y envoie un débit d'air. Une partie de cet air est utilisée pour la combustion, l'autre partie est directement mélangée aux produits de la combustion. La combustion échauffe une barre de métal qui se dilate en fonction de la température. Cette dilatation est prise en compte par un système qui régule le débit d'air d'alimentation de façon à maintenir la barre de métal à une longueur constante donc à une température constante. Ce débit d'air va donc varier selon la chaleur apportée par le gaz et sa mesure permet d'accéder directement à la quantité d'énergie dégagée. L'appareil peut fonctionner à pression constante ou à débit constant. Dans le cas du dispositif de comptage objet de l'invention, le mode d'injection n'importe pas car c'est le même volume brut qui est prélevé à chaque cycle. La précision de mesure n'est donc liée, pour la partie calorimétrique, qu'à la mesure de débit d'air. La capacité de l'échantillonneur (3) peut varier de 0,1 à 1 litre pour des débits d'analyse de 10 à 200 litres par heure aux conditions normales.

Un calculateur est couplé en permanence à l'ensemble de comptage objet du dispositif. Il a pour fonctions de gérer les électrovannes (6) et (7) de l'échantillonneur, de prendre en compte les impulsions données par le compteur de volume (2), de prendre en compte le signal délivré par l'analyseur (4), de calculer l'énergie de chaque séquence et cumuler les énergies horaires , journalières, mensuelles, et de gérer l'étalonnage du système. Il comporte notamment des entrées/sorties pour les fins de courses (25) et (26), pour les électrovannes (6) et (7), une entrée 4-20 mA pour le signal délivré par l'analyseur (4) et, une entrée pour les impulsions délivrées par le compteur (2). Les entrées-sorties se font notamment par un index totalisateur de volumes en façade, une imprimante et un clavier permettant de modifier s'il y a lieu les paramètres de calcul, de lancer le processus d'étalonnage, de régler les échelles des entrées, d'accéder aux valeurs de certaines variables et de les afficher sur imprimante.

Le déroulement d'un cycle N du dispositif est le suivant :
- un volume $v_E$, volume brut de gaz à la pression $P_N$ et à la température $T_N$ est prélevé dans l'échantillonneur (3);
- simultanément un volume $v_E$, volume brut prélevé à $P_{N-1}$ et $T_{N-1}$ au cycle N-1, est poussé vers l'analyseur (4);
- l'analyseur mesure la quantité d'énergie $e_N$ correspondant à celle du volume $v_E$ prélevé au cycle précédent;
- le compteur (2) mesure le volume brut $V_N$ à $P_N$ et $T_N$ transitant dans la conduite.

Une fois le cycle N + 1 achevé, le calculateur calcule la quantité d'énergie livrée pendant la séquence N, à savoir $E_N = K \times V_N \times e_{N+1}$, formule dans laquelle K est un coefficient tenant compte des caractéristiques du système et de la courbe d'erreur du compteur. L'analyseur (4) mesure en fait des valeurs de quantités de chaleur correspondant au pouvoir calorifique inférieur. Pour passer aux valeurs correspondant au pouvoir calorifique supérieur, il suffit de multiplier les résultats obtenus par 1,11. Une étude des différents types de gaz circulant dans les réseaux de distribution montre en effet que l'erreur introduite par cette approximation n'entraîne pas une erreur supérieure à 0,2%

Afin d'apprécier les résultats obtenus grâce au dispositif objet de l'invention, deux dispositifs de comptage ont été installés en parallèle sur un site de livraison de gaz. Une des installations de comptage a été réalisée selon le dispositif de l'invention, l'autre a été constitué d'un comptage de volume sur le site, complété par la mesure des caractéristiques du gaz dans un laboratoire situé sur le réseau en amont, l'ensemble servant de référence. Le comptage de référence était un comptage par orifice équipé de transducteurs de pression différentielle "ROSEMOUNT 1151", d'un transducteur de pression Rosemount et d'une sonde de température associée à une calculatrice "MECI CDN 44" effectuant le calcul de débit. Le laboratoire de mesure n'étant pas implanté directement sur le site, des temps de transit ont été calculés pour l'affectation des caractéristiques du gaz à son passage à la station. Ces temps n'excédaient pas quelques heures. L'incertitude des mesures sur cette chaîne de référence peut être estimée à 2%, compte tenu de l'erreur sur le comptage de volume et de l'imprécision sur les temps de transit.

Les énergies transitées pendant les sept mois où l'expérimentation a été poursuivie, ont varié entre 1.500.000 et 2.400.000 kWh/jour. Les écarts moyens mensuels ressortent du tableau suivant :

| Mois | Ecart moyen mensuel | Ecart type |
|---|---|---|
| Septembre 1989 | -0,51 | 0,62 |
| Octobre 1989 | 0,61 | 0,96 |
| Novembre 1989 | 1,51 | 0,31 |
| Décembre 1989 | 1,12 | 0,30 |
| Janvier 1990 | 0,28 | 0,34 |
| Février 1990 | 0,55 | 1,55 |
| Mars 1990 | -1,49 | 1,20 |

On notera que l'écart moyen mensuel se situe à l'intérieur de la plage d'incertitude de la chaîne de référence.

L'augmentation de l'écart type à partir du mois de Février correspond à un moins bon fonctionnement de l'analyseur dû au fait qu'aucune maintenance ne lui a été apportée. Une maintenance après quatre mois de fonctionnement aurait permis d'éviter cette dérive qui de toute façon n'a pas eu pour effet de faire sortir les mesures de la plage des 2%. On peut donc estimer que le dispositif selon l'invention a donné des résultats équivalents en qualité à une chaîne de référence dont l'installation et l'exploitation sont beaucoup plus onéreuses.

**Revendications**

1. Dispositif de comptage des quantités d'énergie fournies par un réseau de distribution (1) de gaz combustible comportant, sur une conduite, au point de livraison un organe de comptage (2) du volume brut de gaz livré, un échantillonneur (3) qui prélève en permanence, aux conditions de température et de pression de la conduite, des volumes de gaz déterminés, un analyseur (4) qui mesure les éléments nécessaires à la détermination des quantités d'énergie correspondant auxdits volumes et un calculateur (5) qui ramène en permanence lesdites quantités aux volumes bruts livrés pendant le même temps caractérisé en ce que l'échantillonneur (3) se compose d'un cylindre (20) pourvu d'un piston (21) séparant ledit cylindre en deux zones (23) et (24), une zone étant alimentée par le réseau (1) tandis que l'autre alimente l'analyseur (4) et de deux électrovannes trois voies (5) et (6) commandées par le déplacement du piston (21) et qui assurent les mises en communication des zones (23) et (24) alternativement avec le réseau (1) et avec l'analyseur (4).

2. Dispositif suivant la revendication 1 caractérisé en ce que l'échantillonneur (3), placé à proximité du compteur volumétrique (2) et sur la même tuyauterie, est enrobé d'un calorifuge permettant au gaz prélevé de garder la même température que dans la conduite principale.

3. Dispositif suivant la revendication 1 ou 2, caractérisé en ce que le cylindre (20) de l'échantillonneur (3) est placé à l'intérieur de la conduite principale (1).

**Claims**

1. Device for metering quantities of energy supplied by a fuel-gas distribution network (1) comprising, on a pipe, at the delivery point, a member (2) for metering the uncorrected volume of gas supplied, a sampler (3) which continuously takes predetermined volumes of gas at the temperature and pressure conditions of the pipe, an analyzer (4) which measures the factors necessary for determining the quantities of energy corresponding to the said volumes, and a calculator (5) which constantly corrects the said quantities for the uncorrected volumes supplied during the same time, characterized in that the sampler (3) consists of a cylinder (20) provided with a piston (21) separating the said cylinder into two regions (23) and (24), one region being supplied by the network (1) whilst the other supplies the analyzer (4), and two three-way electromagnetic valves (6) and (7) which are operated by the displacement of the piston (21) and which put the regions (23) and (24) alternately into communication with the network (1) and with the analyzer (4).

2. Device according to Claim 1, characterized in that the sampler (3), which is disposed near the volumetric meter (2) and in the same pipe system, is covered with a heat-insulator allowing the gas taken to retain the same temperature as in the main pipe.

3. Device according to Claim 1 or Claim 2, characterized in that the cylinder (2) of the sampler (3) is disposed inside the main pipe (1).

**Patentansprüche**

1. Vorrichtung zum Messen der von einem Brenngasverteilungsnetz (1) bereitgestellten Energiemengen, die in einer Leitung an der Abgabestelle
   - ein Organ (2) zum Messen des Bruttovolumens des gelieferten Gases,
   - einen Probenehmer (3), der unter den Temperatur- und Leitungsdruckbedingungen permanent bestimmte Gasvolumina entnimmt,
   - einen Analysator (4), der die Elemente mißt, die zur Bestimmung der den Volumina entsprechenden Energiemengen notwendig sind, und
   - einen Rechner (5) aufweist, der während der gleichen Zeit permanent die Mengen auf die gelieferten Bruttovolumina zurückführt,
   dadurch gekennzeichnet, daß der Probenehmer (3)
   - aus einem Zylinder (20), der mit einem Kolben (21) versehen ist, welcher den Zylinder in zwei Bereiche (23) und (24) teilt, wobei ein Bereich durch das Netz (1) und der andere durch den Analysator (4) versorgt wird, und
   - aus zwei Drei-Wege-Magnetventilen (6) und (7) besteht, die durch die Verschiebung des Kolbens (21) gesteuert werden und die die Verbindung der Bereiche (23) und (24) mit dem Netz (1) bzw. mit dem Analysator (4) herstellen.

2. Vorrichtung nach Anspruch 1, dadurch gekennzeichnet, daß der Probenehmer (3), der in der Nähe des Volumenmessers (2) und in dem gleichen Rohrsystem positioniert ist, mit einer Wärmeisolation ummantelt ist, die es ermöglicht, daß das entnommene Gas die gleiche Temperatur wie in der Hauptleitung beibehält.

3. Vorrichtung nach Anspruch 1 oder 2, dadurch gekennzeichnet, daß der Zylinder (20) des Probenehmers (3) innerhalb der Hauptleitung (1) positioniert ist.

FIG. 1

FIG. 2